# NEW EUROPEAN PATENT SPECIFICATION

(11) **EP 0 148 025 B2**
(45) Date of publication and mention of the opposition decision: **21.05.1997**
(45) Mention of the grant of the patent: 04.09.1991
(21) Application number: 84309085.3
(22) Date of filing: 27.12.1984
(51) Int. Cl.: A61K 39/40, A61K 9/68

(54) **Antibodies and antibody-containing compositions for inhibiting periodontitis**
Antikörper und Antikörper enthaltende Zubereitungen für die Inhibition von Periodontitis
Anticorps et compositions contenant un anticorps pour l'inhibition de la périodontite

(30) Priority: 29.12.1983 JP 251975/83
(43) Date of publication of application: 10.07.1985
(73) Proprietor: Hashimoto, Takashi, Chofu-shi, Tokyo-to (JP)
(72) Inventor: Tsurumizu, Takashi, Ichikawa-shi Chiba-ken (JP); Hashimoto, Takashi, Chofu-shi Tokyo-to (JP); Sato, Makoto, Hachiman-cho Tokushima-shi Tokushima-ken (JP)
(74) Representative: Hildyard, Edward Martin

(56) References cited:
- GB-A- 2 151 923
- INFECTION AND IMMUNITY, vol. 41, no. 1, July 1983, pages 19-27; J.J. ZAMBON et al.: "Serology of oral actinobacillus actinomycetemcomitans and serotype distribution in human periodontal disease"
- BIOLOGICAL ABSTRACTS/RRM, vol. 26, 1984, abstract no. 79362; K.T. MIYASAKI et al.: "Purification of a serotype defining antigen from actinobacillus-actinomycetemcomitans", & JOURNAL OF DENTAL RESEARCH, 1983, vol. 62, no. SPEC. ISSUE, page 278
- BIOLOGICAL ABSTRACTS/RRM, vol. 23, 1982, abstract no. 66489; J.W. VINCENT et al.: "Human precipitating antibody to eubacterium-brachy and other gram positive oral microorganisms", & JOURNAL OF DENTAL RESEARCH, 1982, vol. 61, no. SPEC. ISSUE, page 310
- Infection and Immunity, 1982, vol. 36, No. 3, pp. 1217-1222; Revis, G. J. et al.

## Description

The present invention relates to antibodies and antibody-containing compositions for preventing or at least reducing (hereinafter referred to as inhibiting) periodontitis.

The term "periodontitis" as used in this specification denotes a wide variety of diseases of humans and animals which occur in the periodontal area of the oral cavity (for example, gingiva, cementum and alveolar bone) induced or aggravated by the action of certain oral bacteria. Periodontitis may be classified as marginal periodontitis, for example, pyorrhoea alveolaris, or apical periodontitis. The biological activities of periodontitis-inducing oral bacteria include the formation of dental plaque on the surface of teeth and the formation of cysts in the periodontal area. Such virulent oral bacteria are exemplified by Actinomyces viscosus, Actinomyces naeslundi, Actinobacillus actinomycetemcomitans and Bacteroides gingivalis. Among them, Actinomyces viscosus is believed to be most important as this microorganism produces a large amount of levan-type polysaccharide from sucrose, which adheres and builds up on the surfaces of teeth and mucous membranes and does harm to the periodontal tissues. Other known periodontitis-inducing oral bacteria are generally weaker periodontitis inducers than Actinomyces viscosus. However, it is well known that these microorganisms when in coexistence in a mixed population with Actinomyces viscosus are liable to exacerbate periodontitis considerably. It has also been observed that the above-mentioned virulent oral bacteria such as Actinomyces viscosus are generally present largely in the focus of periodontitis.

Although various means of treating periodontitis are known which give good results, antibodies capable of inhibiting periodontitis have not previously been reported.

It was previously known that certain oral bacteria capable of inducing periodontitis have pili-like structures (fimbriae) on the cell surface by means of which they adhere to the surfaces of teeth and mucous membranes. However the aetiology of such pili-like structures in periodontitis, and more particularly their antigenic characteristics have never been clarified. The present invention is based upon the discovery that antibodies specific for antigens isolated from the pili-like structures of various oral bacteria capable of inducing or aggravating periodontitis can be used to effectively inhibit periodontitis.

According to one feature of the present invention, there is provided a process for the preparation of antibodies for inhibiting periodontitis, which comprises isolating at least one antigen from the pili-like structures (fimbriae) of at least one microorganism which is capable of inducing or aggravating periodontitis, wherein said isolation comprises suspending said microorganism in a hypertonic buffer solution whereby said antigen is extracted, immunizing a non-human mammal with said at least one antigen and recovering resultant antibodies from said non-human mammal.

In order to administer antibodies of the present invention to humans and animals simply and effectively, the present invention further provides compositions for inhibiting periodontitis, which comprise at least one antibody of the present invention as active ingredient in association with a pharmaceutically acceptable carrier or excipient.

It has been found that by administration of the antibodies of the present invention to humans and animals, the adherence of periodontitis-inducing oral bacteria, at least those of the same species as the bacteria used for the preparation of the antibodies, may be inhibited. It is generally difficult, however, to inhibit the growth of periodontitis-inducing microorganisms and the formation of levan-type polysaccharide using such antibodies. When periodontitis-inducing microorganisms in the oral cavity are inhibited from adhering to the surfaces of teeth they coagulate, for example in the saliva. Coagulated microbial cells can be readily removed from the oral cavity in conventional manner, for example, by the use of usual dentifrices, cleansing agents or gargles. As a result of various experiments using humans and hamsters, it has been found that the virulent periodontitis-inducing oral microorganisms may be removed from the oral cavity by continuous administration, for example for several weeks, of an effective amount of antibodies prepared by the process of the invention, in particular, formulated into a composition as hereinbefore defined.

The term "pili component antigen" used in this specification denotes an antigen isolated from the pili-like structures (fimbriae) on the cell surface of microorganisms capable of inducing or aggravating periodontitis, and which possess said structures. Periodontitis-inducing microorganisms preferred for the process of the invention include Actinomyces viscosus, Actinomyces naeslundi, Bacteroides gingivalis and Actinobacillus actinomycetemcomitans. It is possible to use mutant strains of the above-mentioned species which are capable of inducing or aggravating periodontitis and have the pili-like structures on their cell surface.

As previously stated, Actinomyces viscosus is believed to be the most important of the oral periodontitis-inducing microorganisms. However, it is generally advantageous to use at least one antigen originating from the pili-like structures of a virulent periodontitis-inducing microorganism other than a strain of Actinomyces viscosus, in combination with a pili-component antigen derived from Actinomyces viscosus.

It is preferred to use as the source of the desired antigens periodontitis-inducing bacilli having a particularly high ability to adhere to teeth and/or mucous membranes in the oral cavity. Such microorganisms may readily be selected with reference to, for example, a high ability to adhere to a tube wall in vitro or a high periodontitis-inducing potential in the oral cavity of hamsters.

In the examples and experiments described hereinafter, Actinomyces viscosus Mutant Strain K-TL + and Bacteroides gingivalis of K-Bg-ml were used. These microorganisms were deposited at The Fermentation Research Institute Agency of Industrial Science and Technology in Japan on the 24th April 1982 under FERM BP-411 and FERM BP-410, respectively.

Actinomyces viscosus Mutant Strain K-TL + was obtained by mutation of a corresponding wild strain in conventional manner. When this mutant strain is cultured using, for example, TYC agar plate medium, a large amount of insoluble levan-type polysaccharide is produced from sucrose. If culturing is carried out using a liquid medium, a large number of cells adhere to the tube wall. When Actinomyces viscosus Mutant strain K-TL + is subcultured over an extended period of time or treated with known mutagens, for example, nitrosoguanidine, nitrogen mustard or ultraviolet light, reversion to its parent stain or generation of any other mutant may not be observed. The strain can therefore be considered to be substantially genetically stable. On administration to hamsters, Actinomyces viscosus mutant strain K-TL + adheres well to the surfaces of teeth and mucous membranes in the oral cavity and, if the animals are fed a cariogenic diet, a high ability to induce periodontitis is found with reference to the resorption of the alveolar bone.

Bacteroides gingivalis of Strain K-Bg-ml is a wild strain obtained by selection from wild strains isolated from human periodontitis with reference to a higher ability to react with haemoagglutinin and a higher ability to adhere to the surfaces of teeth. When compared with various other wild strains of the same species, this strain is characterized by its higher ability to adhere to the epithelial cells of mucous membranes in the oral cavity as well as by its higher reactivity with haemoagglutinin. When Bacteroides gingivalis of Strain K-Bg-ml is orally administered to hamsters, its adhering ability is not always very high. However, if hamsters are infected with Actinomyces viscosus Mutant Strain K-TL + and subsequently Bacteroides gingivalis of Strain K-Bg-ml is also orally administered to the same animals either immediately before or after the beginning of the symptons of periodontitis, it is observed that Bacteroides gingivalis of Strain K-Bg-ml adheres well. Moreover, the symptons of the resultant periodontitis may be considerably worse in comparison with the case of sole infection by Actinomyces viscosus Mutant Strain K-TL +.

The mycological characteristics of the above-mentioned two strains are as follows:-
(A) Actinomyces viscosus Mutant Strain K-TL + (FERM BP-411):
I. Morphology: Gram-positive bacillus. 1 x 3-4 um.
II. Growth property: anaerobic (facultative aerobic).
III. Growth on various media:
(1) TYC agar plate medium (pH about 7.2; Stoppelaar et al., Arch. Oral Biol., 12, 1199-1201 (1967)-]:
   Colonies are rough, raised, grainy, mucoid, white and solid, and are covered with abundant insoluble polysaccharides. Margins are irregular.
(2) Brain-heart infusion agar medium (pH about 7.4; in a dish of 9cm diameter; commercial product of Baltimore Biological Laboratories, Inc., U.S.A., hereinafter referred to as BBL):
   Colonies are round, colourless, thick and transparent, and have uniform and smooth surfaces. Margins are irregular.
(3) Tryptocase Soy Broth (pH about 7.8; BBL): Growing from the lower layer of the medium.
(4) Todd Heuwitt Broth (pH about 7.8; BBL): Growing from the lower layer of the medium.

IV. Physiological characteristics:
(1) Production of levan-type polysaccharides: + + +
(2) Ability to adhere to tube wall: + + +
(3) Production of pigment: negative
(4) Growth range: pH 6 to 8.5; temp. 22 to 39°C.
(5) Decomposition of sugars:

Adonite, arabinose, dextrin: negative; fructose, galactose, glucose: positive; xylose, inulin, lactose: negative; maltose, mannitol, mellibiose, raffinose, sucrose: negative.
V. Miscellaneous:

| | |
|---|---|
| (1) Catalase | positive |
| (2) Indole | negative |
| (3) Reduction of nitrate | positive |
| (4) Production of hydrogen sulfide | positive |
| (5) Liquefication of gelatin | negative |
| (6) Hydrolysis of esculin | positive |
| (7) Ability to adhere to the epithelial cells of the mucous membrane in the oral cavity:++ | |

VI. Ability to infect animals:
Induction of periodontitis: + + + (determined with reference to the resorption of alveolar bone.
(B) Bacteroides gingivalis of K-Bg-ml (FERM BP-410):
I. Morphology: Gram-positive bacillus, 0.5 x 0.8 um
II. Growth property: Obligatory anaerobic
III. Growth on various media:
   (1) 10% blood agar plate medium (pH about 7.3; BBL): Colonies are round, large, flat, somewhat shiny and black and have smooth surfaces and margins. No haemolysis.
   (2) Bacteroides agar plate medium (pH about 7.2; in a dish of 9 cm diameter; commercial product of Nissui Seiyaku K.K., Japan):
      Colonies are round, somewhat smaller, flat, uniform and transparent. Surfaces and margins are smooth.
   (3) Tryptocase Soy Broth (pH about 7.8; BBL): Growing from the lower layer of the medium.
IV. Physiological characteristics:
   (1) Formation of pigment: negative.
   (2) Growth range: pH 5.0-8.5; temp. 22 to 39°C.
   (3) Decomposition of sugars:

   Adonite, arabinose, dextrin, fructose, galactose, glucose, inulin, lactose, mannitol, mannose, mellibiose, raffinose, sucrose: all negative.
V. Miscellaneous:
   (1) Mobility: negative
   (2) Production of indole: positive
   (3) Reduction of nitrate: negative
   (4) Production of hydrogen sulfide: positive
   (5) Liquefication of gelatin: positive
   (6) Hydrolysis of esculin: negative
   (7) Ability to adhere to the epithelial cells of the mucous membrane in the oral cavity: + + +
   (8) Haemoagglutination: + + +
VI. Ability to infect animals:
   Induction of periodontitis: ± (determined with reference to the resorption of alveolar bone).

Both Actinobacillus actinomycetemcomitans and Actinomyces naeslundi which may be used for the purpose of the present invention are known per se and are described, for example, in the catalogue of The American Type Culture Collection, 14th edition, pages 26-27 (1980).

The microorganisms which may be used for the purpose of the present invention may be cultured in conventional manner used for culturing various microorganisms of the same species. Thus, the culturing may be effected by the use of both organic and synthetic media, although liquid media may be preferred for mass propagation. For example, culturing may usually be effected at pH 5.0-8.5 (for example, about 7.4) and at a temperature of 22 to 39°C (for example about 37°C) under anaerobic conditions and continued, for example, for 24-72 hours. Examples of preferred media are as follows:-

### Medium A

Tryptocase peptone (1.7%, BBL), phyton peptone (0.3%, BBL), yeast extract (0.5%, Difco., U.S.A.), potassium phosphate, dibasic (0.25%), sodium chloride (0.5%) and glucose (0.25%); pH about 7.4.

### Medium B

Medium A further containing hemine (0.007%).

### Medium C

Medium A containing hemine (0.005%) and vitamin K (0.0001%).
Medium A is suitable for culturing microorganisms of the genus Actinomyces. Media B and C are suitable for culturing microorganisms of the genus Bacteroides.

A pili-component antigen solution suitable for immunizing a non-human mammal according to the present invention may be prepared, for example, in the following manner.

A seed culture is prepared by culturing a suitable strain at a temperture of 35-37°C and a pH of 6.5-7.5 for 24-48 hours. The resulting seed culture is transferred to a main medium having the same composition as that of the seed medium and cultured for 48-72 hours under similar conditions. After completion of culturing, the number of living cells in the culture broth is usually at least about 10⁸ cells/ml. The cells are separated, for example, by centrifugation (8000 r.p.m./20 min) and suspended in a suitable buffer solution conventionally used to suspend such cells for example, 0.1-1M acetic acid;sodium chloride buffered solution (pH about 6.8-8.0) or 0.01-0.75M phosphate buffered 1M sodium chloride solution (pH about 6.5-8.0). If desired, a suitable non-ionic surfactant such as, for example, Triton X-100 (RTM) (0.001-0.01%; commercial product of Rohm & Haas Co., U.S.A.) may be added. The solution is then treated with, for example, ultrasonic waves (e.g. 10-20 KHz/5-10 min) to extract the desired antigen.

The desired antigen may be fractionated and purified by using, for example, one or more of the following techniques: column chromatography, the isoelectric point (focussing) precipitation method, the fractional precipitation method using cold solvent such as ethanol, the membrane concentration method and the salting-out method using ammonium sulfate.

If desired, the resultant antigen solution may be treated with a suitable inactivating agent, for example, formalin (0.2-0.02%), followed by dialysis against a similar buffer solution.

The antigen solution thus obtained is then diluted with a suitable buffer solution, for example, 0.1-1M phosphate-buffered 0.9% sodium chloride solution (pH 6.2-7.0) to adjust the protein N concentration to 5-50 µg/ml. To the diluted solution, aluminium hydroxide is added as adjuvant to adsorb the antigen, the final concentration of aluminium being preferably adjusted to about 100-500 µg/ml. Instead of aluminium hydroxide, it is possible to use an equal amount of Freund's complete adjuvant. To the resultant antigen solution, a suitable antiseptic agent, for example, thiomerosal (0.005-0.1% w/v) may, if desired, be added.

Instead of treating the cell suspension with ultrasonic waves, ammonium sulfate may be added (about 20-70%, for example, 30%). The solution is agitated to dissolve the ammonium sulfate and is then allowed to stand at a low temperature, for example, at 4°C, for 24-48 hours until the desired antigen is precipitated. The supernatant is collected and the precipitated fraction is suspended densely in a suitable buffer solution such as, for example, 0.05-0.5M phosphate-buffered 1M sodium chloride solution (pH about 7.0-8.0). The cell suspension is dialyzed against a similar buffer solution at a low temperature, for example, at 4°C. The residual solution is centrifuged (for example, 8000 r.p.m./20 min) and then fractionated and purified in a similar manner to that described above.

The antigen solution thus-obtained for immunizing non-human mammals may be preserved over an extended period of time at a low temperature, for example, below 10°C.

In order to avoid denaturation of the pili-component antigen, it is preferred to carry out the extraction, isolation and purification of the desired antigen at a low temperature, for example below 10°C.

The pili-component antigen which may be isolated from Actinomyces viscosus Mutant Strain K-TL + by the methods given above has been subjected to quantitative determination of protein (by Hartree's method, using bovine serum albumin as reference) and carbohydrate (by the phenol/sulphuric acid method, using D-glucose as reference), and its molecular weight has been determined by gel filtration using Sephadex G-100 (RTM) (Pharmacia Fine Chemicals AB., Sweden) with reference to phosphorylase b, bovine serum albumin, egg albumin, chymotrypsinogen and other proteins having known molecular weights. The results are as follows:-
(1) The pili-component antigen is an acidic glycoprotein containing about 15-25% (e.g. about 20%) carbohydrate and about 75-85% (e.g. about 80%) protein and having a molecular weight of about 5-8 x 10⁴ (e.g. about 55000).
(2) Polyacrylamide disc electrophoresis of the pili-component antigen results in a characteristic broad band towards the cathode.
(3) A pI value for the pili-component antigen of not more than 3.5 is obtained by the isoelectric focussing method of Versterberg et al., using 1% of the carrier ampholite (pH 3.5-10.0; commercial product of LKB Produkter AB., Sweden).
(4) If sucrose density-gradient ultracentrifugation is carried out with the pili-component antigen, the antigen is found in the fractions having a sucrose density of about 11-22% and a specific gravity of about 1.4-1.6.
(5) If gel filtration of a crude pili-component antigen solution is carried out using Sephacryl S-300 (RTM) or Sephadex G-100 (RTM) (Pharmacia Fine Chemicals AB., Sweden), two peaks are eluted (monitored by absorbance at 280 nm). The pili-component antigen is recovered from the first peak, which is positive in the reaction with human or rabbit serum.

Similar results were obtained using pili-component antigens isolated from Actinomyces naeslundi, Bacterioides gingivalis and Actinobacillus actinomycetemcomitans.

If desired, a non-human mammal may be immunized with two or more pili-component antigen solutions originating from different microbial strains capable of inducing or aggravating periodontitis. The antigen solutions may be combined or used separately for immunization. It is possible, for example, to mix a pili-component antigen solution originating from a periodontitis - inducing Actinomyces viscosus strain with another pili-component antigen solution originating from a periodontitis - inducing Bacteroides gingivalis strain and immunize a non-human mammal with the resultant mixture. Alternatively, it is possible to use the same antigen solutions separately.

Non-human Mammals suitable for immunization, include, mice, rats, rabbits, guinea pigs, goats, horses and cattle. The dose of the antigen may vary, depending upon various factors, for example, the type of mammal and the particular pili-component antigen. However, usually it is possible to use an antigen in an amount of, for example, 10-500 µg for smaller animals and 100-2000 µg for larger animals once daily (calculated as protein N), and the immunization may be effected preferably by injection, for example, 2-5 times with an interval of 2-5 weeks. If desired, it is possible to immunize a non-human mammal by oral administration of a pili-component antigen solution in an amount of, for example, 5-20 fold the amount generally used for injection and the administration may be continued for example, for 3-12 days. For instance, in the case of immunizing a rabbit, a pili-component antigen solution originating from Actinomyces viscosus (containing 100-300 µg of antigen, calculated as protein N) may be injected 2-5 times with an interval of 2-5 weeks. If desired, it is possible to use one or more pili-component antigens originating from Bacteroides gingivalis, Actinomyces viscosus, Actinomyces naeslundi, and Actinobacillus actinomycetemcomitans in combination. For example, about 2 weeks after final immunization, blood is collected from the mammal, and plasma containing the desired antibodies may be obtained in conventional manner. If desired, the resultant plasma may further be purified to obtain an antiserum in conventional manner.

It is believed that antibodies are formed in the body of the mammal by administration of the desired antigen in the usual manner.

The resultant plasma or antiserum may be preserved over an extended period of time at a low temperture, preferably by freeze-drying.

Although it is possible to administer antibodies of the present invention directly to humans and animals, the present invention further provides compositions which comprise an effective amount of at least one antibody of the present invention in association with a pharmaceutically acceptable carrier or excipient by means of which it is possible to administer the desired antibody or antibodies simply and more effectively.

Compositions of the present invention may be in the form of a solid, semi-solid or liquid suitable for oral administration, and thus may take the form of powders, syrups, capsules, ampoules, granules, tablets, suspensions, emulsions, drops or lozenges. Suitable excipients include, lactose, potato or soluble starch, and magnesium stearate, and suitable carriers are exemplified by sodium chloride solution, almond oil and drinks containing lactic acid-producing bacteria such as yogurt and fruit juices.

Compositions of the present invention may further contain, for example, bonding agents, stabilizers, emulsifiers, suspending agents, preservatives, antiseptic agents and various other additives, conventionally used in the art. Advantageously, a composition may be in the form of, for example, a dentifrice, gargle, chewing gum, icecream, or confectionery.

In order to administer a given amount of an antibody or antibodies of the present invention more easily and effectively, a composition may be formulated, for example, as tablets, ampoules, capsules, lozenges and various other dosage unit forms known in the art. Such a dosage unit form may contain antibodies of the present invention in such an amount that each antibody may be administered to humans at a daily dose of, for example, 2-4 units (as hereinafter defined). It has been found that by continuous daily administration to humans of a composition of the present invention, for example for several weeks, such that the daily dose of each antibody is, for example, 2-4 units, various oral microorganisms capable of inducing periodontitis of at least the same species as the microorganisms used for the preparation of the antibodies may be eliminated from the oral flora. When a larger amount of at least one antibody was continuously administered orally to hamsters for a long period of time, for example, for 6-12 months, no abnormality was noted by pathological examination.

The unit of the antibody titre is expressed by the precipitation value obtained by the double diffusion method with reference to Goldman et al. [Isolation and Characterization of Glial Filaments from Human Brain, J. Cell Biol. 78, 426 (1978)].

In the following non-limiting examples and experiments, which illustrate the invention, the culturing was effected at 37°C under anaerobic conditions and commercially available media were used at their specified pHs, unless otherwise stated.

### Example 1

### Preparation of Actinomyces viscosus Mutant Strain K-TL + (FERM BP-411):

Wild strains were collected from the focus of human periodontitis and identified as Actinomyces viscosus with reference to their mycological and serological characteristics. The strains were cultured at 37°C for 24 hours by using Tryptocase Soy Broth [pH 7.3; 40 ml; BBL., U.S.A.]. The cells were separated from the culture broth by centrifugation (8000 r.p.m./20 min) and washed three times by centrifugation (each 8000 r.p.m./20 min) using 0.75M phosphate-buffered sodium chloride solution (pH 7.0; each 200 ml). The cells were suspended in 0.75M phosphate-buffered sodium chloride solution (pH 7.0; 10 ml) containing 0.2% nitrogen mustard and were kept at 37°C until more than 90% of the cells were killed (for about 60-90 min). The cell suspension was washed three times by centrifugation effected in a similar manner to that described above by the use of 0. 75M phosphate-buffered sodium chloride solution (pH 7.0; each 200 ml). The surviving cells were smeared on a TYC agar plate medium (pH 7.4; 5 ml in a dish of 9 cm diameter) for culturing at 37°C for 48 hours to form colonies. The culture was allowed to stand at room temperture for 24 hours. The resultant colonies were selected to obtain solid colonies having irregular margins and covered with abundant insoluble polysaccharides. The above-mentioned procedure may be repeated until a desired colony is obtained. The resultant colony was collected and cultured to obtain the desired mutant strain.

### Example 2

### Preparation of Bacteroides gingivalis of Strain K-Bg-ml (FERM BP-410):

Wild strains collected from the focus of human pyrrhoea alveolaris were identified as Bacteroides gingivalis and selected with reference to a higher reactivity with haemoagglutinin and a higher ability to adhere to the epithelial cells of the oral mucous membrane. The selected strain was cultured at 37°C for 72 hours by using 10% blood agar plate medium [pH about 7.3; 18 ml in a dish of 9 cm diameter; BBL, U.S.A.]. The resultant colonies were selected and each colony was cultured at 37°C for 72-96 hours by using Tryptocase Soy Broth [20 ml]. On each occasion, the microtitre plate method was used to dilute the culture broth stepwise (x 2) with a physiological solution of sodium chloride (pH 7.0) to which (0.025 ml) was added 0.5% sheep red cell suspension (0.025 ml) with stirring. The resultant mixture was allowed to stand at room temperature for 60-120 minutes. Where the red cells spread over the entire surface on the bottom of the vessel, the cells were regarded as positive to the agglutination reaction. In this manner, a sample of cells exhibiting the highest reactivity at the highest dilution was selected and cultured. The above-mentioned procedure may, if desired, be repeated until a desired strain is obtained. When subcultured over an extended period of time using various known media, the characteristics of the resultant strain were found to be genetically stable.

### Example 3

(1) Preparation of a pili-component antigen solution:
   Actinomyces viscosus Mutant Strain K-TL + (FERM BP-411) was cultured using Medium A as hereinbefore defined (100 ml) at 37°C for 24 hours to obtain a seed which was transferred to a main medium (Medium A; 15000 ml) for culturing at 37°C for 24 hours. To the culture broth was added crystals of ammonium sulfate to give a saturation of 33%. The mixture was allowed to stand at 4°C overnight, followed by centrifugation (8000 r.p.m./20 min) to separate the cells. The separated cells were suspended in 0.1M phosphate-buffered 1M sodium chloride solution (pH 7.0-8.0; 150 ml) with agitation (1-5 rotations per min) at 4°C for about 72 hours. The cell suspension was centrifuged (8000 r.p.m./30 min) to separate the cells. To the supernatant was added ammonium sulfate at a saturation of 60%. Ammonium sulfate was dissolved with stirring, and the solution was allowed to stand at 4°C for 48 hours to precipitate the desired antigen. After removal of the supernatant, the precipitated fraction was recovered by centrifugation (5000 r.p.m./10 min) and suspended in a similar phosphate buffered 1M sodium chloride solution (100 ml). The cell suspension was put into a cellophane tube and dialyzed against a similar phosphate-buffered 1M sodium chloride solution (more than 5000 ml) at 4°C for 48 hours to remove ammonium sulfate and dialyseable impurities. The residual solution was collected and centrifuged (8000 r.p.m./30 min).
   About 240 ml of the supernatant was collected and diluted with a similar phosphate-buffered 1M sodium chloride solution to give a protein N concentration of 100 ug per ml. The diluted solution (200 ml) was subjected to sucrose density-gradient ultracentrifugation using a Hitachi 65P ultracentrifuge equipped with a zonal rotor RP 235T (commercial product of Hitachi Limited, Tokyo; sucrose density 5-30%; 35000 r.p.m./18 hours). It was found that the desired antigen was present in the fractions having a sucrose density of about 11-22% and its protein N was about 40 µg/ml. The resultant extracted solution containing the desired antigen was put into a cellophane tube, to which polyvinylpyrrolidone was applied to reduce the amount to 1/10. The concentrated solution was then diluted with 0.75M phosphate-buffered sodium chloride solution (pH 6.2-6.5) to give a protein N concentration of 50-100 µg per ml. An equal amount of Freund's complete adjuvant was added to the diluted solution to obtain an antigen solution suitable for immunization of a non-human mammal.
(2) The resultant antigen solution (0.1 ml) was administered to a rabbit having a body weight of about 3 kg in conventional manner by injection on the back of the animal. 4 weeks after this, a similar treatment was carried out using the same amount of antigen. 4 weeks after the secondary immunization, blood was collected from the animal in conventional manner to prepare a serum. Ammonium sulfate was added to the separated serum at a saturation of 33% and dissolved with stirring. The mixture was allowed to stand at 4°C for 48 hours to obtain a precipitate. The precipitate was collected by centrifugation (8000 r.p.m./20 min) and put into a cellophane tube for dialysis at 4°C against purified water to remove ammonium sulfate completely. The residual material was freeze-dried to obtain the desired antibodies.

### Example 4

Bacteroides gingivalis Strain K-Bg-ml (FERM BP-410) was cultured in a similar manner to that described in Example 3 at 37°C for 24 hours using Medium C as hereinbefore defined. The culture broth was centrifuged (8000 r.p.m./20 min) to separate the cells which were then suspended in 0.1M phosphate-buffered 1M sodium chloride solution (pH 8.0; 150 ml). The cell suspension was gently agitated at 4°C with 1-5 rotations per minute, followed by centrifugation (8000 r.p.m./20 min) to remove the cells. In this manner, an extracted solution (120 ml) was prepared. To the supernatant were added small portions of 10% zinc sulfate solution to a final concentration of 1%. The pH of the solution was adjusted to 6.0 with 10% sodium carbonate solution and the solution was left at 4°C for 24 hours to form a precipitate. After removal of the supernatant, the precipitate was collected by centrifugation (5000 r.p.m./5 min). To the collected material were added crystals of disodium phosphate (12 H₂O; 150 g). The mixture was well-mixed and filtered using a glass filter under suction. The material was washed with 10% disodium phosphate (about 30 ml) and was then filtered under reduced pressure to remove the resultant zinc phosphate. In this manner, a second extracted solution (about 95 ml) was obtained.

The first and second extracted solutions were combined together (about 215 ml). To the combined solutions was added saturated ammonium sulfate solution to give a saturation of 60%, and the mixture was allowed to stand at 4°C for 24 hours to form a precipitate. After removal of the supernatant, the precipitate was collected by centrifugation (5000 r.p.m./5 min) and was then dissolved in 0.1M phosphate-buffered 1M sodium chloride solution (pH 8.0; 10 ml). A cellophane tube was used for dialysis of the solution at 4°C for more than 24 hours against a similar buffer solution (more than 2000 ml). The residual solution was centrifuged (8000 r.p.m./30 min). The resulting supernatant (about 45 ml) contained protein N in an amount of about 22 mg/ml and was purified and concentrated in a similar manner to that described in Example 3.

On carrying out sucrose-density gradient ultracentrifugation, the desired antigen was present in the fractions having a sucrose density of 16-22% and exhibitng a haemoagglutination activity of more than 1:14800. The resultant extracted solution containing the desired antigen was diluted with 0.75M phosphate-buffered sodium chloride solution (pH 6.2-6.5) to a protein N concentration of 100-200 µg/ml). A Freund's complete adjuvant containing antigen solution was prepared in a similar manner to that described in Example 3. The pili-component antigen solution was used to immunize a rabbit to obtain the desired antibodies.

### Example 5

Actinobacillus actinomycetemcomitans isolated from the focus of human periodontitis was treated in a similar manner described in Example 3 to obtain a pili-component antigen as follows:- The strain was cultured at 37°C in Medium C (15000 ml) for 24 hours. To the resultant culture broth were added crystals of ammonium sulfate at a saturation of 33%. The mixture was allowed to stand at 4°C for 24 hours, followed by centrifugation (8000 r.p.m./20 min) to separate the cells. The separated cells were suspended in 0.1M phosphate-buffered 1M sodium chloride solution (150 ml; pH 8.0) to extract the active material. Then ammonium sulfate was added to the cell suspension at a saturation of 60% to form a precipitate which was collected by centrifugation (5000 r.p.m./10 min). The supernatant was dialyzed using a cellophane tube against a similar phosphate-buffered 1M sodium chloride solution (more than 5000 ml). The residual solution was centrifuged (8000 r.p.m./30 min) to obtain a supernatant (180 ml) which was then diluted with a similar phosphate-buffered 1M sodium chloride solution to give a protein N concentration of 200 ug/ml. The diluted solution (200 ml) was subjected to sucrose density gradient ultracentrifugation and the desired antigen was recovered in the fractions having a sucrose density of about 12-15%. The protein N concentration of the resultant antigen solution was about 14 mg per ml.

In a similar manner to that described in Example 3, the antigen soluion was diluted with 0. 75M phosphate-buffered sodium chloride solution (pH 6.2-6.5) to a protein N concentration of 100 µg/ml and mixed with an equal amount of Freund's complete adjuvant to obtain an antigen solution for immunizing a non-human mammal. A rabbit was immunized in conventional manner by the use of the antigen solution thus prepared. Blood was collected from the rabbit in conventional manner, from which a serum was separated. The serum was fractionated and purified by adding 33% ammonium sulfate to obtain the desired antibodies.

In Example 6 et seq. described hereinafter, the compositions contained a portion of at least one antibody isolate selected from the antibody isolates described in Examples 3, 4 and 5. The antibody titre of each isolate was adjusted to about 2-4 units of the gel precipitation value determined by the double diffusion precipitation method.

### Example 6

Lozenges were prepared in conventional manner by the use of glucose (1 g), antibody isolate (0.05 ml) and soluble starch (0.05 g).

### Example 7

Syrup was prepared in conventional manner by mixing together CMC-Na (sodium carboxymethyl cellulose; 0.2 g), 20% fructose solution (20 ml), ethylparaffin (0.04 g) and antibody isolate (0.1 ml).

### Example 8

Dental paste was prepared in conventional manner by mixing together calcium hydrogen phosphate (fine powder; 60%), glycerol (30%), CMC-Na (10%) and Parabens (antiseptic agent; 0.25%) and adding to the mixture a portion of at least one antibody isolate. The titre of antibodies derived from each isolate was adjusted to 4 units per 50 g of the product.

### Example 9

Corn syrup (25 g) and recitin (0.2 g) were added to gum base (20 g) at 95°C. The mixture was well-mixed at 80°C for 5 minutes. To the mixture were then added fructose (55 g) and citric acid (0.1 g), and the mixture was cooled to 60°C. An antibody isolate (0.05 ml), obtained using a pili-component antigen derived from Actinomyces viscosus, was added to the mixture, which was then used to prepare a chewing gum in conventional manner.

Further chewing gums were prepared as above, but incorporating in addition antibodies corresponding to one or more pili-component antigens originating from Bacteroides gingivalis, Actinobacillus actinomycetemcomitans and Actinomyces naeslundi (0.05 ml of each antibody isolate).

### Experiments

In the following experiments, compositions containing antibodies corresponding to one or more pili-component antigens were used to investigate the ability to inhibit the adherence of periodontitis-inducing microorganisms in the oral cavities of humans and animals. As test animals, Golden hamsters and gerbils (each group consisting of 6 females) were used, unless otherwise specified.

### Experiment 1

Dental paste prepared as in Example 8 and containing an antibody corresponding to a pili-component antigen originating from Actinomyces viscosus was used to investigate the change in the concentration of wild strains of Actinomyces viscosus in the oral flora of humans. Dental plaque was collected 3 times from the oral flora of each member of a test group consisting of 20 adults (male). On each occasion, the test samples were cultured at 37°C for 24 hours by the use of TYC agar plate medium and propionic acid agar plate medium. 4 members of the test group were found to be hosts of Actinomyces viscosus. Every day after breakfast and dinner, dental paste was applied by the hosts of Actinomyces viscosus. The method of application was not specified. Dental plaque was collected from the oral flora of these males at least once per week. The test samples were cultured in a similar manner to that described above to investigate the change in the concentration of Actinomyces viscosus. As shown in the following Table 1, wild strains of Actinomyces viscosus could hardly be detected in samples collected from 2 of the males after about 2-3 weeks and in the samples collected from the remaining 2 males after about 4 weeks.

**Table 1**

| | Administration period (week) | | | | | |
|---|---|---|---|---|---|---|
| | 0 | 2 | 4 | 6 | 8 | 10 |
| Male 1 | + | - | - | - | - | - |
| Male 2 | + | + | + | - | - | - |
| Male 3 | + | + | + | - | + | - |
| Male 4 | + | - | - | - | - | - |
| + wild strains of Actinomyces viscosus detectable - wild strains of Actinomyces viscosus not detectable or hardly detectable. | | | | | | |

### Experiment 2

As test animals, Golden hamsters (30 days after birth) were used. Two groups were used as test groups and a further two groups were used for control purpose. Actinomyces viscosus Mutant Strain K-TL + (FERM BP-411) described in Example 1 was cultured at 37°C for 24 hours using Medium A as hereinbefore defined to obtain a culture broth containing about 10⁸ living cells/ml. 0.1 ml of the culture broth was orally administered once daily for 5 days to each animal. The microorganism adhered to the teeth of all the animals. After this, Bacteroides gingivalis Strain K-Bg-ml (FERM BP-410) was cultured at 37°C for 24 hours using Medium C as hereinbefore defined to obtain a culture broth containing about 10⁸ living cells/ml. 0.1 ml of the resultant broth was orally administered once daily for 5 days to each animal (35 days after birth) of the 2nd and 4th groups. 0.1 g of an appropriate dental paste prepared as in Example 8 was applied once daily for 14 days from the 40th day after birth to each animal of the first and second groups using a dental brush (Lumident S; commercial product of Bayer Japan Dental K.K., Japan). During the test period, all animals were fed with a cariogenic diet (Diet 2000; Funabashi Nojo, Japan) and deionized water ad libitum. The test was continued for 30 days after the beginning of the use of the dental paste. From time to time during the test period, samples were collected from the oral cavity of each animal and cultured at 37°C for 72 hours using TYC agar plate medium, propionic acid agar medium and 10% blood agar plate medium to investigate the adherence of the virulent strains. It was found that use of the dental paste of the present invention resulted in gradual decrease of the concentration of the virulent strains in the oral flora of each animal of the first and second groups. The virulent strains thus disappeared from the oral flora of a proportion of the test animals within one week and from the remaining test animals within two weeks after the beginning of the use of the dental paste. In the oral cavity of each animal of the control groups, no decrease in the concentration of Actinomyces viscosus was found, while the concentration of Bacteroides gingivalis appeared to decrease gradually.

After completion of the test period, each animal of the test groups was anaesthetized with pentabarbital. The maxilla was collected from each animal and treated in an autoclave at 118-121°C for 1-2 minutes. The soft part was removed from the maxilla which was then well-washed with water and treated with 20% silver nitrate solution for 5 minutes. The maxilla was then dried to obtain a sample of the teeth. The difference in the resorption of alveolar bone between the animals of the test groups and the animals of the control groups was investigated with reference to, in particular, the resorption of alveolar bone in the area of the first molar of the lower jaw using the method of Tsukiyama et al. [Journal of Dental Health, Japanese Version, Vol. 28. No. 3, page 149 (1978)]. The results obtained are shown in the following Table 2.

**TABLE 2**

| Group | Degree of resorption of alveolar bone (mean value) | | |
|---|---|---|---|
| | Mean Body weight (g) | Upper jaw | Lower jaw |
| 1 | 108 | 33.5 | 45.4 |
| 2 | 114 | 29.6 | 51.5 |
| 3 | 119 | 98.7 | 100 |
| 4 | 110 | 87.6 | 97.4 |

### Experiment 3

As test animals, gerbils (20 days after birth) were used. One group was used as a test group and a second group as the control group. Actinobacillus actinomycetemcomitans (wild strain) used in Example 5 was cultured at 37°C for 24 hours using Medium C as hereinbefore defined to obtain a culture broth containing about 10⁸ living cells per ml. 0.1 ml of the culture broth was orally administered once daily for 5 days to each animal. The virulent strain adhered in the oral flora of all animals. 30 days after birth, an appropriate dental paste prepared as in Example 8 was applied to the molar surfaces of the animals of the test group in a similar manner to that described in Experiment 2. From time to time, test samples were collected from the oral cavity of all animals. Each sample was cultured at 37°C for 72 hours using 10% blood agar plate medium to investigate the adherence of the virulent bacteria in the oral cavity. As shown in the following Table 3, it was found that use of the dental paste of the present invention resulted in rapid decrease of the concentration of the virulent strain in the oral cavity of the test animals so that the strain disappeared substantially within a week after the beginning of application of dental paste. The concentration of the virulent strain decreased in the oral cavity of the control animals, but much more slowly.

**TABLE 3**

| Group | No. of Animals | Mean Body weight increase (%) | Infected animals | | | | |
|---|---|---|---|---|---|---|---|
| | | | 25 | 30 | 45 | 50 | * |
| Test | 6 | 35 | 5 | 2 | 0 | 0 | |
| | | | 83 | 33 | | | (%) |
| Control | 6 | 34.6 | 6 | 6 | 4 | 1 | |
| | | | 100 | 100 | 66.7 | 17 | (%) |

| | | | | | | | |
|---|---|---|---|---|---|---|---|
| * days after birth | | | | | | | |

### Experiment 4

Chewing gum prepared as in Example 9 and containing antibodies corresponding to a pili-component antigen derived from Actinomyces viscosus together with other appropriate antibodies corresponding to pili-component antigens of different origin, was administered orally to 10 adults (5 male and 5 female) daily at bedtime for several weeks. Each member of the test group at the start of the experiment hid at least one strain of Actinobicillus actinomycetemcomitans, Actinomyces naselundi or Bacteroides gingivalis, in addition to at least one strain of Actinomyces viscosus, in their oral cavity. Each member kept the chewing gum in their oral cavity for as long as possible (>20 minutes), while chewing at intervals.

The results were at least equal or superior to the results obtained in Example 1. Within about 4 weeks after the beginning of the experiment, no oral bacteria of the above-mentioned species could be detected in the oral cavities of members of the test group.

## Claims

1. A process for the preparation of antibodies for inhibiting periodontitis, which comprises isolating at least one antigen from the pili-like structures (fimbriae) of at least one microorganism which is capable of inducing or aggravating periodontitis, wherein said isolation comprises suspending said microorganism in a hypertonic buffer solution whereby said antigen is extracted, immunizing a non-human mammal with said at least one antigen and recovering resultant antibodies from said non-human mammal.

2. A process as claimed in claim 1 wherein the hypertonic buffer solution contains sodium chloride.

3. A process as claimed in claim 2 wherein the sodium chloride is at a concentration of about 1M.

4. A process as claimed in any one of claims 1 to 3, wherein said microorganism is of the genus Actinomyces.

5. A process as claiyed in claim 4, wherein said microorganism is the strain Actinomyces viscosus.

6. A process as claimed in claim 5, wherein said microorganism is Actinomyces viscosus mutant strain K-TL+ (FERM BP-411) or a mutant strain thereof capable of inducing or aggravating periodontitis.

7. A process as claimed in claim 4, wherein said microorganism is a strain of Actinomyces naeslundi.

8. A process as claimed in any one of claims 1 to 3, wherein said microorganism is selected from the genus Bacteroides and/or the genus Actinobacillus.

9. A process as claimead in claim 8, wherein said microorganism is selected from Bacteroides gingivalis and/or Actinobacillus actinomycetemcomitans.

10. A process as claimed in claim 9, wherein said microorganism is Bacteroides gingivalis Strain K-Bg-ml (FERM BP-410), or a mutant thereof capable of inducing or aggravating periodontitis.

11. A process as claimed in any one of the preceding claims wherein said at least one antigen comprises an acidic glycoprotein containing about 15-25% carbohydrate and about 75-85% protein, and having a molecular weight of about 5 -8 x 10⁴ (determined by gel filtration) and a specific gravity of about 1.4-1.6 (determined by sucrose density gradient ultracentrifugation).

12. A composition for inhibiting periodontitis wherein the active-ingredient comprises at least one antibody specific for an antigen from the pili-like structures (fimbriae) of at least one microorganism which is capable of inducing or aggravating periodontitis in association with a pharmaceutically acceptable carrier or excipient.

13. A composition as claimed in claim 12 in a form suitable for oral administration.

14. A composition as claimed in claim 13 in a form selected from chewing gum, ice cream, syrup, confectionery and drinks containing lactic acid-producing bacteria.

15. A composition as claimed in claim 13 in a form selected from capsules, ampoules, tablets and lozenges.

16. A composition as claimed in claim 13 in a form selected from dentifrices and gargles.

17. The mutant strain of Actinomyces viscosus designated Actinomyces viscosus Mutant Strain K-TL+ (FERM BP-411) and mutants thereof which are capable of inducing or aggravating periodontitis.

18. A substantially microbiologically pure culture of Bacteroides gingivalis Strain K-BG-ml (FERM BP-410) and mutants thereof which are capable of inducing or aggravating periodontitis.

19. Antibodies specific for any antigen obtainable from the pili-like structures (fimbriae) of Actinomyces viscosus Mutant Strain K-TL+ (FERM BP-411) or mutants thereof which are capable of inducing or aggravating periodontitis.

## Patentansprüche

1. Verfahren zur Herstellung von Antikörpern für die Inhibition von Periodontitis aufweisend, daß man mindestens ein Antigen aus den Pili-ähnlichen Strukturen (Fimbrien) mindestens eines Mikroorganismus, der Periodontitis verursachen oder verschlimmern kann, isoliert, wobei die Isolation Suspendieren des Mikroorganismus in einer hypertonischen Pufferlösung, wobei das Antigen extrahiert wird, Immunisieren eines nicht-menschlichen Säugetiers mit wenigstens einen Antigen und Rückgewinnung der resulierenden Antikörper von dem nicht-menschlichen Säugetier umfaßt.

2. Verfahren nach Anspruch 1, wobei die hypertonische Pufferlösung Natriumchlorid enthält.

3. Verfahren nach Anspruch 2, wobei die Konzentration an Natriumchlorid etwa 1 M beträgt.

4. Verfahren nach einem der Ansprüche 1 bis 3, wobei der Mikroorganismus der Gattung Actinomyces angehört.

5. Verfahren nach Anspruch 4, wobei der Mikroorganismus der Stamm Actinomyces viscosus ist.

6. Verfahren nach Anspruch 5, wobei der Mikroorganismus der Actinomyces viscosus-Mutantenstamm K-TL+ (FERM BP-411) oder ein Mutantenstamm davon ist, der Periodontitis verursachen oder verschlimmern kann.

7. Verfahren nach Anspruch 4, wobei der Mikroorganismus ein Stamm der Actinomyces naeslundi ist.

8. Verfahren nach einem der Ansprüche 1 bis 3, wobei der Mikroorganismus aus der Gattung Bacteroides und/oder der Gattung Actinobacillus ausgewählt ist.

9. Verfahren nach Anspruch 8, wobei der Mikroorganismus aus Bacteroides gingivalis und/oder Actinobacillus actinomycetemcomitans ausgewählt ist.

10. Verfahren nach Anspruch 9, wobei der Mikroorganismus der Bacteroides gingivalis-Stamm K-Bg-ml (FERM BP-410) oder eine Mutante davon ist, die Periodontitis verursachen oder verschlimmern kann.

11. Verfahren nach einem der vorhergehenden Ansprüche, wobei das zumindest eine Antigen ein saures Glykoprotein mit etwa 15-25 % Kohlenhydrat und etwa 75-85 % Protein und einem Molekulargewicht von etwa 5-8 x 10⁴ (bestimmt durch Gelfiltration) und einer relativen Dichte von etwa 1,4-1,6 (bestimmt durch Rohrzucker-Dichtegradienten-Ultrazentrifugation) enthält.

12. Zubereitung für die Inhibition von Periodontitis, wobei der wirksame Bestandteil mindestens einen Antikörper unfaßt, der spezifisch für ein Antigen aus den Pili-ähnlichen Strukturen (Fimbrien) mindestens eines Mikroorganismus ist, der Periodontitis in Verbindung mit einen pharmazeutisch geeigneten Träger oder Bindemittel verursachen oder verschlimmern kann.

13. Zubereitung nach Anspruch 12 in einer für die orale Verabreichung geeigneten Form.

14. Zubereitung nach Anspruch 13 in Form von Kaugummi, Eiscreme, Sirup, Süßigkeiten oder Milchsäure produzierende Bakterien enthaltenden Getränken.

15. Zubereitung nach Anspruch 13 in Form von Kapseln, Ampullen, Tabletten oder Pastillen.

16. Zubereitung nach Anspruch 13 in Form von Zahnputzmitteln oder Gurgelmitteln.

17. Mutantenstamm von Actinomyces viscosus, bezeichnet als Actinomyces viscosus-Mutantenstamm K-TL+ (FERM BP-411), und Mutanten davon, die Periodontitis verursachen oder verschlimmern können.

18. Mikrobiologisch im wesentlichen reine Kultur des Bacteroides gingivalis-Stammes K-Bg-ml (FERM BP-410) und Mutanten davon, die Periodontitis verursachen oder verschlimmern können.

19. Antikörper, spezifisch für jedes Antigen, das aus den Pili-ähnlichen Strukturen (Fimbrien) des Actinomyces viscosus-Mutantenstammes K-TL+ (FERM BP-411) oder Mutanten davon, die Periodontitis verursachen oder verschlimmern können, erhältlich ist.

## Revendications

1. Procédé de préparation d'anticorps pour inhiber la périodontite, qui consiste à isoler au moins un antigène des structures filamenteuses (fimbriae) d'au moins un micro-organisme qui est capable d'induire ou d'aggraver la périodontite, ledit isolement comprenant la mise en suspension dudit micro-organisme dans une solution tampon hypertonique pour extraire ledit antigène, à immuniser un mammifère non humain avec au moins ledit antigène et à recueillir dudit mammifère non humain les anticorps résultants.

2. Procédé suivant la revendication 1, dans lequel la solution tampon hypertonique contient du chlorure de sodium.

3. Procédé suivant la revendication 2, dans lequel le chlorure de sodium est à une concentration d'environ 1M.

4. Procédé suivant l'une quelconque des revendications 1 à 3, dans lequel est utilisé un micro-organisme du genre Actinomyces.

5. Procédé suivant la revendication 4, dans lequel est utilisée une souche d'Actinomyces viscosus.

6. Procédé suivant la revendication 5, dans lequel est utilisée la souche mutante K-TL+ (FERM BP-411) d'Actinomyces viscosus ou un mutant de cette souche capable d'induire ou d'aggraver la périodontite.

7. Procédé suivant la revendication 4, dans lequel est utilisée une souche d'Actinomyces naeslundi.

8. Procédé suivant l'une quelconque des revendications 1 à 3, dans lequel est utilisé un micro-organisme choisi dans le genre Bacteroides et/ou dans le genre Actinobacillus.

9. Procédé suivant la revendication 8, dans lequel est utilisé un micro-organisme choisi entre Bacteroides gingivalis et/ou Actinobacillus actinomycetemcomitans.

10. Procédé suivant la revendication 9, dans lequel est utilisée la souche K-Bg-ml (FERM BP-410) de Bacteroides gingivalis ou un mutant de cette souche capable d'induire ou d'aggraver la périodontite.

11. Procédé suivant l'une quelconque des revendications précédentes, dans lequel l'antigène en question dont au moins un est présent comprend une glycoprotéine acide contenant environ 15 à 25 % de glucide et environ 75 à 85 % de protéine, et ayant un poids moléculaire d'environ 5-8 x 10⁴ (déterminé par filtration sur gel) et une densité d'environ 1,4 à 1,6 (déterminée par ultra-centrifugation dans un gradient de densité de saccharose).

12. Composition pour inhiber la périodontite, dans laquelle l'ingrédient actif comprend au moins un anticorps spécifique d'un antigène des structures filamenteuses (fimbriae) d'au moins un micro-organisme qui est capable d'induire ou d'aggraver la périodondite, en association avec un support ou excipient acceptable du point de vue pharmaceutique.

13. Composition suivant la revendication 12, sous une forme qui convient pour l'administration orale.

14. Composition suivant la revendication 13, sous une forme choisie entre une gomme à mâcher, une crème glacée, un sirop, un produit de confiserie et des boissons contenant des bactéries productrices d'acide lactique.

15. Composition suivant la revendication 13, sous une forme choisie entre des gélules, des ampoules, des comprimés et des pastilles.

16. Composition suivant la revendication 13, sous une forme choisie entre des dentifrices et des gargarismes.

17. Souche mutante d'Actinomyces viscosus appelée souche mutante K-TL+ (FERM BP-411) d'Actinomyces viscosus et ses mutants qui sont capables d'induire ou d'aggraver la périodontite.

18. Culture pratiquement pure du point de vue microbiologique de la souche K-Bg-ml (FERM-BP 410) de Bacteroides gingivalis et de ses mutants qui sont capables d'induire ou d'aggraver la périodontite.

19. Anticorps spécifiques de tout antigène pouvant être obtenu à partir de structures filamenteuses (fimbriae) de la souche mutante K-TL+ (FERM BP-411) d'Actinomyces viscosus ou de ses mutants qui sont capables d'induire ou d'aggraver la périodontite.
